# FASCICULE DE BREVET EUROPEEN

(11) **EP 1 189 855 B1**
(45) Date de publication et mention de la délivrance du brevet: **25.08.2004**
(21) Numéro de dépôt: 00949600.1
(22) Date de dépôt: 30.06.2000
(51) Int. Cl.: C07B 39/00, C07C 201/12

(54) **UTILISATION DE NITRILES COMME SOLVANTS APROTIQUES POLAIRES**
VERWENDUNG VON NITRILEN ALS APROTISCHE POLARE LÖSUNGSMITTEL
USE OF NITRILES AS POLAR APROTIC SOLVENTS

(30) Priorité: 02.07.1999 FR 9908558
(43) Date de publication de la demande: 27.03.2002
(73) Titulaire: RHODIA CHIMIE, 92512 Boulogne Billancourt Cedex (FR)
(72) Inventeur: LAURAIN, Nathalie, F-69540 Irigny (FR); ZARD, Samir, F-91190 Gif-sur-Yvette (FR)
(74) Mandataire: Ricalens, François
(86) Numéro de dépôt international: PCT/FR2000/001864
(87) Numéro de publication internationale: WO 2001/002320

(56) Documents cités:
- DE-A- 4 342 284
- US-A- 4 684 734
- H. SUZUKI: "Synthesis of 3,4-difluorobenzonitrile and monofluorobenzonitriles by means of halogen-exchange fluorination" JOURNAL OF FLUORINE CHEMISTRY, vol. 52, no. 3, mai 1991 (1991-05), pages 341-351, XP002131599 LAUSANNE CH
- DATABASE WPI Section Ch, Week 8630 Derwent Publications Ltd., London, GB; Class E14, AN 1986-193369 XP002148129 & JP 61 126042 A (ASAHI GLASS), 13 juin 1986 (1986-06-13)

## Description

La présente invention a pour objet l'utilisation, comme solvants aprotiques polaires, de nitriles aliphatiques de poids moléculaires relativement élevé.

Elle a plus particulièrement pour objet l'utilisation de ces nitriles comme solvants dans des réactions de substitution nucléophile, en particulier, de substitution nucléophile aromatique.

La présente invention concerne également de nouveaux réactifs utiles pour l'échange entre le fluor et des halogènes ou des halogènes conduisant à la fluoration d'une molécule.

La plupart des solvants aprotiques polaires sont des solvants coûteux, d'accès difficile et requièrent des conditions de stockage d'emploi relativement contraignantes.

Ainsi, dans le cas particulier de l'échange chlore/fluor, l'un des solvants les plus efficaces est le sulfolane qui est coûteux et qui nécessite avant l'emploi des mesures de purification, notamment pour éviter qu'il ne contienne trop d'eau.

En outre, le sulfolane n'est pas réputé particulièrement sain.

Le problème est particulièrement aigu dans le cas de l'utilisation de ces solvants lors de la synthèse des dérivés fluorés. Ces dérivés sont de plus en plus utilisés dans l'industrie pharmaceutique ou agrochimique en raison de leurs propriétés qui s'écartent notablement des propriétés des dérivés halogénés usuels.

Les dérivés fluorés sont difficiles d'accès, notamment en raison de la réactivité du fluor qui est telle qu'il n'est pas possible d'obtenir directement des dérivés fluorés d'une manière aisée.

Aussi, une des techniques les plus employées pour fabriquer les dérivés fluorés consiste à faire réagir un dérivé halogéné, ou pseudo-halogéné, en général chloré pour échanger l'halogène avec un fluor mineral en général, un fluorure de métal alcalin. Ce dernier étant avantageusement de poids atomique élevé (en général, au moins égal à celui du potassium).

A l'échelle industrielle, le fluorure le plus utilisé est le fluorure de potassium qui constitue un compromis économique satisfaisant entre coût et efficacité.

Cette technique est utilisée aussi bien pour l'échange sur des carbones de nature aliphatique (c'est-à-dire des carbones dont l'hybridation est sp³) que pour les carbones aromatiques ou vinyliques (carbones d'hybridation sp²).

Cette technique d'échange d'halogène est surtout utilisée pour les échanges sur des noyaux aromatiques, qu'ils soient homocycliques ou hétérocycliques.

L'échange dans le cas du chlore et du fluor, comme plus généralement le cas des Substitutions Nucléophiles Aromatiques (SN_{Ar}), est le plus souvent extrêmement lent et difficile, sauf dans les cas exceptionnels où le noyau aromatique est extrêmement déprimé en électrons. Par ailleurs, la réaction est extrêmement sensible aux impuretés et notamment à l'eau, laquelle conduit à la formation d'éther aromatique en lieu et place des dérivés fluorés attendus.

Cette réaction d'éthérification n'est pas la seule réaction parasite, car les produits obtenus sont souvent des produits fragiles. Cette fragilité s'étend au complexe dit «de Meisenheimer», qui est propre aux réactions de substitution nudéophile dites «aromatiques».

En outre, brochant sur le tout, la réaction doit être menée à des températures élevées (c'est-à-dire nettement supérieures à 100°C), souvent au voisinage de 200°C, ce qui implique l'utilisation de solvants et de catalyseurs résistant bien à ces températures.

C'est pourquoi un des buts de la présente invention est de fournir une technique qui conduise à l'utilisation de réactifs ou de solvants moins sophistiqués que ceux précédemment utilisés.

Un autre but de la présente invention est de fournir une technique qui, appliquée aux fluorures alcalins, voire aux fluorures alcalino-terreux, permette d'augmenter significativement leur cinétique de réaction.

Un autre but de la présente invention est de fournir une technique qui permette d'augmenter les rendements de la réaction. Ces rendements sont essentiellement le rendement de réaction, ou RR, et le rendement de transformation, ou RT, qui est l'image de la sélectivité de la réaction.

Un autre but de la présente invention, est de fournir des solvants d'accès facile, peu sophistiqués et de bonne résistance chimique et thermique.

Un autre but de la présente invention est de fournir des solvants, qui facilitent les réactions d'échange entre halogènes et/ou pseudo-halogènes et notamment les réactions de substitution nucléophile SN₁, SN₂, et surtout SN_{Ar}.

Un autre but de la présente invention est de fournir des réactifs susceptibles de promouvoir ou de réaliser les échanges par substitution nucléophile, surtout les substitutions nucléophiles d'ordre 2 et les substitutions nucléophiles aromatiques.

Ces buts et d'autres qui apparaîtront par la suite sont atteints au moyen de l'utilisation comme solvant aprotique polaire dans les réactions de substitution nucléophile d'un nitrile dont la masse moléculaire est supérieure à 79, de préférence supérieure à 90 et par le fait que ledit nitrile comporte au moins une fonction nitrile portée par un carbone aliphatique (c'est-à-dire d'hybridation sp³) avec la condition que lorsque les carbones aliphatiques portent, outre une fonction nitrile, une fonction électroattractrice dont la constante de Hammett σₚ est supérieure ou égale à 0,05, il ne porte pas d'hydrogène.

Cette utilisation comme solvant aprotique polaire est particulièrement intéressante dans le cas des réactions de substitution nucléophile, notamment de substitution nucléophile aromatique.
Le nitrile est avantageusement un composé comportant au moins une fonction nitrile de nature aliphatique, c'est-à-dire que le carbone porteur de la ou d'une fonction nitrile est d'hybridation sp³.

Il est souhaitable que lorsque le carbone porteur de la fonction nitrile est porteur d'une autre fonction électro-attractrice, c'est à dire d'une fonction dont la constante de Hammett σₚ est supérieure à 0,2, le carbone ne porte pas d'hydrogène. Il est préférable que cette contrainte s'applique dès lors que la constante de Hammett σₚ est supérieure à 0.

Pour plus de détails sur les constantes de Hammett on peut se référer à la troisième édition du manuel écrit par Monsieur le Pr. Jerry March "advanced organic chemistry" (pages 242 à 250) et édité par John Wiley and sons.

Il est souhaitable que le nitrile constituant le solvant, ou une partie du solvant, soit tel que par le chemin le plus direct, deux fonctions électroattractrices (en général des fonctions nitrile) soient séparées par au moins deux carbones, avantageusement trois carbones, de préférence quatre carbones. C'est notamment le cas lorsque le nitrile est un dinitrile. Notamment dans le cas où les fonctions électroattractrices sont séparées par seulement deux, voire trois carbones, il est préférable que le ou les carbones porteurs des fonctions électroattractrices et notamment de la fonction nitrile, ou des fonctions nitriles, portent un groupement électrodonneur, par exemple du type alcoyle (pris dans son sens étymologique d'un alcool dont on a enlevé la fonction OH), y compris aralcoyle.

Lorsque le nitrile est un polynitrile aliphatique, un effet significatif est déjà obtenu quand au moins un des carbones porteurs de la fonction nitrile porte une fonction ou un groupe électrodonneur. Cela peut être placé sur une échelle par le rapport entre les hydrogènes en alpha et les hydrogènes au total.

Bien entendu parmi la population des carbones porteurs de fonction nitrile, plus grande est la proportion de carbone également porteur de fonction électrodonneuse, meilleure est la sélectivité. Toutefois la cinétique devient alors moins favorable.

Il est préférable que les groupes électrodonneurs soient choisis parmi ceux qui sont peu encombrant, lorsqu'il s'agit de d'alcoyles, on préfère le méthyle aux alcoyles primaires et ces derniers aux secondaires.

Pour la notion de groupement électroattracteur et électrodonneur, on se reportera au livre de chimie générale Advanced Organic Chemistry Reaction Mecanism and Structure third Edition de Jerry March édité par John Wiley & Sons, notamment au chapitre 9 et plus particulièrement aux pages 242 à 248. Pour juger si un groupe est électroattracteur ou électrodonneur, on regardera le signe de la constante de Hammett telle qu'elle apparaît notamment dans le tableau 4 de la page 244.

Selon la présente invention, il est préférable que les éventuels groupes électro-attracteurs présents sur la molécule de solvant soient moins électro-attracteurs que le groupe nitrile, de préférence au plus égal aux groupes trifluorométhyle, plus préférentiellement à la valeur 0,40.

Les groupements électrodonneurs sont avantageusement des groupes électrodonneurs par effet inductif.

Il est souhaitable que le nombre d'hydrogènes en alpha d'un groupe électroattracteur dont le σₚ est voisin de celui du nitrile, c'est-à-dire dont la constante de Hammett σₚ est au moins égale à 0,40, soit tel que le rapport entre le nombre d'hydrogènes en alpha et le nombre d'hydrogènes total (H_{α}/H_{T}) soit inférieur ou égal à 2/3, avantageusement inférieur ou égal à 1/2, de préférence inférieur ou égal à 40%, plus préférentiellement inférieur ou égal à 30%.

La masse moléculaire rapportée à la fonction nitrile aliphatique est au moins égale à 40, avantageusement à 47, de préférence à 54. Cette valeur est obtenue par la masse moléculaire totale divisée par le nombre de fonctions nitriles aliphatiques, c'est-à-dire le nombre de fonctions nitriles portées par un atome de carbone d'hybridation sp³.

Il est préférable que le point de fusion desdits nitriles soit inférieur à 100°C, de préférence à 50°C plus préférentiellement à 30°C.

Il est préférable que le point d'ébullition dudit nitrile soit au moins égal à 150°C, de préférence à 200°C. Les nitriles peuvent être utilisés seuls ou en mélange avec d'autres solvants. Toutefois, dans ce dernier cas, il est souhaitable soit qu'ils soient mélangés avec d'autres nitriles selon la présente invention, soit qu'ils soient largement majoritaires en masse par rapport aux autres solvants. Ainsi, il est préférable que lorsqu'un nitrile selon l'invention est mélangé à un solvant qui n'est pas un autre nitrile selon la présente invention, que le pourcentage de nitrile en masse par rapport à l'ensemble des solvants soit au moins égal à 50%, de préférence à 2/3, plus préférentiellement à 3/4.

Il est avisé, lorsqu'on utilise d'autres solvants que les nitriles selon la présente invention, d'utiliser des solvants relativement peu polaires. C'est-à-dire des solvants dont la constante diélectrique relative ε est au plus égale à 20, de préférence au plus égale à environ 10 (les 0 ne sont pas considérés comme des chiffres significatifs). Il est également préférable que l'indice donneur défini comme le ΔH, (variation d'enthalpie) exprimé en kilocalories de l'association du dissolvant aprotique dipolaire avec le pentachlorure d'antimoine, soit au plus égal à environ 10.

Comme solvant susceptible d'être mélangé avec les nitriles selon l'invention, on peut citer ceux présentant un point d'ébullition relativement élevé, c'est-à-dire un point d'ébullition au moins égal à 130°C de préférence à 150°C-200°C, ainsi que les dérivés du type chloroaromatique, voire l'anisole.

Il est également préférable que la solubilité de l'eau dans ces solvants susceptibles d'être mélangés aux nitriles selon l'invention, soit aussi faible que possible, de préférence inférieure à 15%, préférentiellement à 10%, préférentiellement à 5%. Il est à noter que certain des nitriles selon l'invention sont susceptibles de dissoudre des quantités significatives d'eau (jusqu'à de l'ordre de 10% voire 20% en masse), mais ils présentent toujours une très faible hygroscopie. Ce qui permet d'éliminer aisément l'eau éventuellement présente comme impureté.
Pour des raisons économiques il est préférable d'utiliser comme nitrile ceux qui ne comportent comme fonction que des fonctions nitriles.

La formule préférée des nitriles selon la présente invention est du type suivant :

A-L-C(R₁)(R₂)-C≡N

dans laquelle R₁ et R₂, semblables ou différents, sont des groupes, avantageusement hydrocarbonés, qui soient donneurs ou faiblement électro-attracteurs c'est-à-dire dont le σₚ est inférieur à 0,10 ou bien un hydrogène (rappelons que l'hydrogène étant le groupe de référence, il n'est ni donneur, ni attracteur, son σₚ est par définition égal à 0).

Avantageusement, R₁ et/ou R₂ sont alcoyle ou hydrogène. Lorsqu'ils sont alcoyle, il est préférable que leur nombre de carbones soit au plus égal à 6, avantageusement, à 4.

L est un bras choisi parmi les simples liaisons ou un groupe alcoylène, éventuellement interrompu par des fonctions éthers issues d'époxydes comme cela est précisé plus loin, et notamment polyméthylène. Il comporte avantageusement au plus 10 atomes de carbone, de préférence au plus 8. L est avantageusement non fonctionnalisé.

A peut être hydrogène, aryle, alcoyle, avantageusement de nombre de carbones au plus égal à 6, A peut également être un groupe :

-C(R₃)(R₄)-CN

où R₃ et R₄ semblables ou différents, ont les mêmes valeurs que R₁ et R₂, et ce avec les mêmes préférences.

Sans que cela soit préféré, Le bras L peut comporter d'autres groupements du type A, mais le nombre de groupements de type A est limité par les contraintes ci dessus et celles relatives aux points de fusion, dudit nitrile. En effet, pour jouer le rôle de solvant, le nitrile doit être fondu et relativement peu visqueux à la température d'usage. il est préférable qu'il y ait au plus 3, avantageusement au plus deux, de préférence au plus 1 seul groupe A.

La viscosité exprimée en millipascal seconde à la température d'usage, doit être au plus égale à 500 mPas, avantageusement au plus égale à 100 mPas, plus préférentiellement à 50 mPas.

Il est préférable que ces valeurs soient atteintes à des températures au plus égales à 150°C, avantageusement à 100°C.

Quoique cela ne soit pas préféré, le groupe L peut comporter plusieurs chaînons de groupe oxyde d'éthylène ou oxyde de propylène, plus précisément ce sont des groupes issus de la polycondensation, plus exactement de l'oligocondensation des oxydes d'éthylène et/ou de propylène.

Le nombre total de carbones des nitriles selon la présente invention est avantageusement au plus égal à 20, de préférence au plus égal à 10.

La présente invention vise aussi des compositions utiles comme réactifs ou comme milieux utiles pour les SN_{Ar}, et notamment pour les échanges entre halogène et notamment entre fluor et halogène de rang atomique plus élevé que le fluor.

Ces compositions comportent comme solvant un nitrile selon la présente invention et comportent en outre un agent de transfert de phase, soit du type cryptant, tel que les éthers couronnes, soit du type onium ou inium. Les oniums sont des composés dont le nom dans la nomenclature comporte comme, en général comme suffixe, la séquence de lettres «onium». Il s'agit des composés de métalloïde, notamment de la colonne de l'azote et de la colonne du soufre, qui sont suffisamment substitués pour porter une charge positive. Ainsi, les atomes de la colonne de l'azote, lorsqu'ils sont substitués quatre fois par un radical hydrocarboné, constituent des oniums. Ainsi, on peut utiliser des ammoniums quaternaires, des phosphoniums quaternaires comme agent de transfert de phase. De même, les sulfoniums (tertiaire dans leur cas) constituent également des agents de transfert de phase, mais ces derniers sont moins intéressants car étant relativement plus fragiles que les autres. Les «iniums», dont l'affixe est «inium», se déduisent des oniums en remplaçant deux groupes hydrocarbonés par un seul groupe hydrocarboné, ce dernier étant alors relié au métalloïde par une double liaison (exemple le pyridinium). Les «oniums» sont préférés aux «iniums».

Les oniums utilisés comme agent de transfert de phase sont connus de l'homme de métier.

Les plus couramment utilisés sont les tétraalcoylammoniums et les tétraalcoyles ou tétraphénylphosphoniums. Ces derniers présentent toutefois le désavantage d'être relativement chers.

Pour éviter les béta élimination, le plus couramment utilisé est le tétraméthylammonium, bien qu'il soit relativement peu stable à partir d'environ 150°C ; on peut également citer le benzyle triméthylammonium. Comme agent de transfert de phase, on peut enfin utiliser les sels de métaux alcalins particulièrement lourds, tels que le césium et le rubidium. Toutefois, ces composés sont relativement toxiques et surtout très coûteux, ce qui limite leur usage

La quantité d'agent de transfert de phase est en général comprise entre 0,1% et 20%, de préférence entre 1 et 5% de la masse de solvant utilisée. La limite supérieure est toutefois facilement franchissable et ne représente qu'un problème de coût. Si on l'exprime par rapport au substrat une valeur comprise entre 0,1 et 10% molaire est des plus courantes.

Ainsi, les oniums sont choisis dans le groupe des cations formés par les éléments des colonnes V B et VI B (telles que définies dans le tableau de la classification périodique des éléments publiés au supplément du bulletin de la Société chimique de France en janvier 1966, avec respectivement quatre ou trois chaînes hydrocarbonées).

Pour réaliser l'échange entre le fluor et les halogènes de rang atomique plus élevé, il convient d'avoir une source de fluorure. Cette source de fluorure peut être des fluorures alcalins ou alcalino-terreux, mais bien plus préférentiellement alcalins. En général, on utilise un fluorure de potassium.

Ainsi, il est souhaitable que le solide en suspension présente une granulométrie tels que son d₉₀ définis en tant que la taille de la maille laissant passer 90% (en masse du solide) est au plus égale à 100 µm, avantageusement au plus égale à 100 µm, de préférence au plus égale à 20 µm. La limite inférieure est avantageusement telle que le d₁₀ dudit solide en suspension est moins égal à 0,1 µm, de préférence au moins égal à 1 µm. il parfois plus simple de ne mesurer que le d₅₀, dans ce cas on peut indiquer que le d₅₀ est avantageusement au plus égal à 20 micromètres.

En général, le rapport entre ledit fluorure alcalin et ledit substrat est compris entre 1 et 1,5, de préférence aux alentours de 1,25 de la stoechiométrie (par rapport aux halogènes à échanger). Ce rapport n'est toutefois en général pas critique dès lors qu'il permet une agitation convenable du réactif. Sauf pour certaine réaction d'échange aliphatique, il est préconisé que teneur en eau du réactif soit au plus égale à environ 2%, de préférence 1% en masse par rapport à la masse du réactif. Comme cela est connu de l'homme de métier pour d'autres solvants, Pour les SN_{Ar} il est préférable d'éliminer aussi complètement que possible l'eau éventuellement présente dans le réactif et de descendre à des valeurs de 1‰, et même de 0,1‰.

La température de l'échange est avantageusement comprise entre 100 et 250°C, de préférence entre 120 et 200°C.

Lorsque les substrats sont volatils, on peut distiller le produit désiré au fur et à mesure de sa formation en utilisant la propriété des dérivés fluorés qui sont dans leur très grande majorité plus volatils que leur homologues chlorés, bromés ou iodés.

Les substrats sont avantageusement des halogénures lourds (c'est-à-dire dont le poids atomique est supérieur à celui du fluor) d'aryle. Pour obtenir de bons rendements, il est préférable d'utiliser des aryles dont la richesse en électrons a été significativement appauvrie. Cet appauvrissement peut être dû soit à la présence dans le cycle aromatique (à six chaînons) d'un hétéroatome comme par exemple dans la pyridine ou la quinoléine.

Bien évidemment, l'appauvrissement électronique peut être provoqué aussi par la présence de groupes électro-attracteurs. La pauvreté en électrons peut être due à ces deux causes. Pour obtenir un bon échange, il est préférable que la somme des σₚ sur un cycle à six chaînons aromatique homocyclique soit au moins égale à 0,4.

Parmi les groupes électro-attracteurs qui, à eux seuls, créent les conditions d'un bon échange, on peut citer les groupes nitro, perfluoroalcoyle, nitrile et les dérivés des fonctions acides. Les substrats les plus intéressants sont les substrats à noyau pyridinique et les substrats à noyau phénylique. Dans les exemples de l'invention, on se sert du dichloronitrobenzène comme paradigme, enseignant par l'exemple les techniques d'échange selon la présente invention, transposables à l'ensemble des substrats aromatiques susceptibles d'être soumis à l'échange du fluor.
Selon un des modes préférés de la présente invention un réalise l'échange, avantageusement l'échange SN_{Ar}, sous l'action de micro-ondes et en présence de césium. La conjonction des micro-ondes, et des solvants nitriles selon la présente invention surtout en présence du césium donne de très bons résultats. Les résultats les meilleurs sont ceux obtenus quand au moins la majeure partie, avantageusement les 2/3, de préférence les 4/5 et même au moins 90% du fluorure est introduite sous la forme de fluorure de césium. Pour obtenir un procédé compétitif, il convient alors de prévoir le retraitement des sels de césium.

En général, la réaction est menée à une température inférieure à celle retenue pour une réaction conventionnelle c'est-à-dire sans l'activation actinique selon la présente invention.

Il est préférable de mener la réaction sous activation actinique à une température d'au moins 10°C, avantageusement 20°C, de préférence 40°C inférieure à celle de la limite de température usuellement admise pour ledit solvant utilisé.

Selon un des modes possibles, voire préféré de la présente invention, les micro-ondes sont émises par périodes courtes (de 10 secondes à 15 min) alternant avec des phases de refroidissement. Les durées respectives des périodes d'émission de micro-ondes et des périodes de refroidissement sont choisies de manière à ce que la température à la fin de chaque période d'émission de micro-ondes demeure inférieure à une température initiale fixée et qui est en général inférieure à celle de la résistance des ingrédients du mélange réactionnel.

Il est également possible de réaliser l'invention selon un mode opératoire dans lequel le mélange réactionnel est soumis simultanément aux micro-ondes et à un refroidissement. Selon cette variante, la puissance dégagée par les micro-ondes est alors choisie de manière à ce que, pour une température initiale fixée, généralement celle de fonctionnement, elle soit équivalente à l'énergie évacuée par le système de refroidissement et ceci à la chaleur dégagée ou absorbée par la réaction près.

Le procédé d'activation revendiqué a, par ailleurs, pour avantage d'être compatible avec un mode de fonctionnement en continu. Ce mode d'utilisation permet avantageusement de s'affranchir des problèmes d'échanges thermiques susceptibles d'être générés lors des opérations d'ouvertures et fermetures du réacteur où sont émises les micro-ondes. Selon ce mode de fonctionnement, les matériaux à activer sont introduits en continu via un orifice d'entrée au sein du réacteur où ils subissent une activation par micro-ondes et on évacue, en continu, dudit réacteur via un orifice de sortie les produits activés.

On peut également procéder à une récupération en continu des composés les plus volatils au fur et à mesure de leur formation. Cette récupération peut être par exemple réalisée par distillation.

Selon un mode privilégié de l'invention, il est préconisé d'utiliser une puissance dégagée par les micro-ondes comprise entre 1 et 50 watts par milliéquivalent d'ions fluorure. De même, les micro-ondes sont de préférence utilisées à une fréquence de 300 MHz à 3 GHz. La fréquence utilisée est généralement de 2,45 GHz et la longueur d'onde associée est voisine de 12 cm dans l'air, la pénétration du champ électromagnétique peut varier entre 2 et 10 cm suivant l'importance des pertes.

Il est également souhaitable de se plier à la contrainte selon laquelle la puissance dégagée par les micro-ondes est comprise entre 2 et 100 watts par gramme de mélange réactionnel.

Ainsi que cela a été mentionné auparavant, la présence d'un catalyseur de transfert de phases est utile, voire nécessaire à la bonne marche de la réaction. Les meilleurs catalyseurs de transfert de phases utilisables sont en général des oniums, c'est-à-dire ce sont des cations organiques dont la charge est supportée par un métalloïde. Cependant quand on utilise le fluorure de césium comme source principale de fluor il n'est pas utile de prévoir d'autres catalyseurs de transfert de phases que le césium.

Au cours de l'étude qui a mené à la présente invention, il a été montré que l'action des micro-ondes sur les oniums et les iniums en présence d'une forte quantité de fluorures était extrêmement néfaste à la survie de ce catalyseur de transfert de phases.

Selon la présente invention, il a été montré que la présence d'anions moins agressifs vis-à-vis des oniums tels que, par exemple, le chlorure, permettait la stabilisation dudit onium.

Ainsi, l'on s'aperçoit, par exemple, qu'au cours d'une réaction d'échange chlore/fluor, la stabilité de l'onium croît avec l'avancement de la réaction puisque cette réaction dégage des anions chlorures.

D'une manière plus générale, il est préférable de s'arranger pour que, au cours de la réaction, la présence d'un anion distinct des fluorures soit assurée en quantités supérieures à une fois, avantageusement à deux fois, de préférence à trois fois la quantité en équivalent dudit onium instable.

Ainsi qu'on l'a mentionné auparavant, l'ion chlorure est un bon candidat pour réduire la dégradation des oniums au cours de la réaction.

Selon un mode privilégié de l'invention, lorsque le catalyseur de transfert de phase est un onium instable en présence de fluorures, la réaction est menée en présence de chlorures en quantité supérieure à une fois la quantité en équivalent dudit onium instable.
Cette instabilité des oniums rend encore plus grand l'intérêt de travailler sans onium mais en utilisant comme seul agent de transfert de phase le césium.

Ainsi qu'on l'a mentionné précédemment, le fluorure, alcalin ou alcalino-terreux est au moins partiellement présent sous la forme d'une phase solide.

Avantageusement, le fluorure est un fluorure d'un métal alcalin de nombre atomique au moins égal à celui du sodium et de préférence est un fluorure de potassium, ou de césium.

Il convient de signaler que parmi les fluorures utilisables, figurent également les fluorures complexes de type KHF₂. Toutefois, on privilégiera l'emploi de fluorures non porteurs d'atome d'hydrogène car ils ne conviennent que rarement aux substitutions aromatiques.

Dans la présente description, on utilise comme exemple paradigmatique des réactions mettant en oeuvre les fluorures alcalins la réaction d'échange chlore/fluor, connue parfois dans le domaine sous l'acronyme anglo-saxon de "halex" (halogen exchange).

Selon la présente invention, il est également possible, lorsque le produit d'arrivée est plus volatil que le substrat et plus volatil que le solvant, d'éliminer au fur et à mesure de leur formation les dérivés visés.

Cela permet d'éviter de trop long temps de séjour dans des conditions particulièrement dures.

En outre, cela permet de jouer sur la loi d'action de masse.

Des exemples qui suivent, l'homme du métier peut inférer que, en terme d'activité, les dinitriles ramifiés donnent de meilleurs résultats que le mononitrile. En sélectivité, le mononitrile est meilleur que le dinitrile ramifié, lui-même meilleur que le dinitrile.

Les exemples qui suivent illustrent l'invention.

### Exemples

### Mode opératoire général des exemples

Dans un réacteur ou un tube Schott placé sous atmosphère d'azote, on charge le fluorure de potassium, le dichloro-2,4-nitrobenzène, le solvant, puis on chauffe à la température indiquée pendant 4 h. A la fin des 4 h, le mélange réactionnel est ensuite filtré et analysé par chromatographie en phase gazeuse.

### Exemple 1

Dans cet exemple, le mode opératoire général a été repris dans les conditions suivantes, dichloronitrobenzène (DCNB ) avec la quantité stoechiométriquement nécessaire de fluorure de potassium pour un double échange (en fait un léger excès égal à 1,05QS), une quantité de chlorure de tétraméthylammonium (TMAC) égale à 0,04 fois la quantité de dichloronitrobenzène exprimée en mole. Les chiffres entre les parenthèses sises avant le nom du produit chimique expriment son rapport molaire en prenant le substrat comme référence.
(1) DCNB + (2) KF + (0,04) TMAC + (3) Solvant / 170°C, 4 h, Tubes Schott

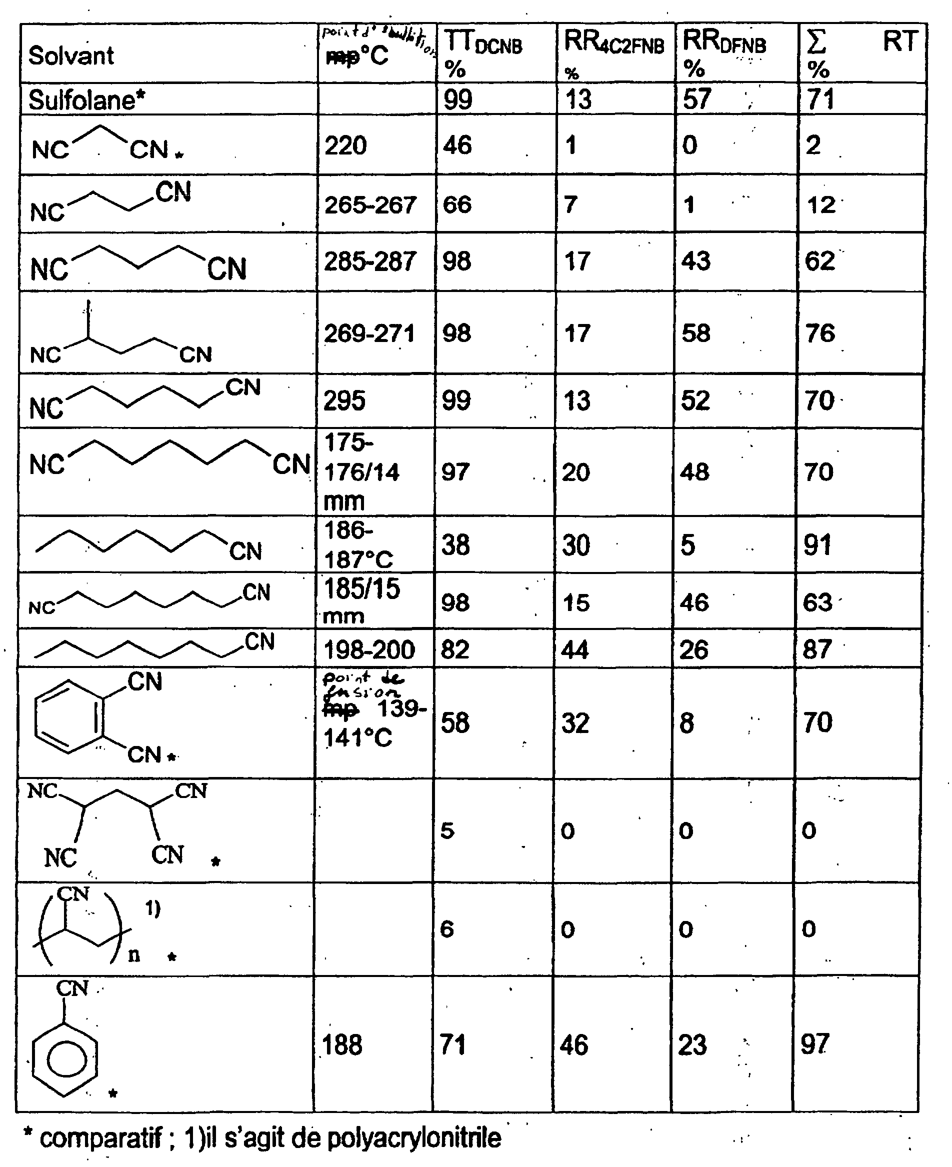

### Exemple 2 - essai sous micro ondes dans l'adiponitrile.

La molécule testée est ici le DCNB avec uniquement du fluorure de césium Caractéristiques de l'équipement utilisé pour le texte :
- le réacteur est en quartz et présente un volume de 40 ml
- l'agitateur est en verre
- la puissance maximum du générateur est 300 W.

La régulation de température est manuelle et non automatique ; la puissance du micro-onde est ajustée manuellement en fonction du temps pour maintenir la température constante. Tous les composés sont introduits à l'ambiante ; le micro-onde est mis en route et fonctionne à plein (P = 300 W) jusqu'à l'obtention de la température désirée. On règle alors la puissance du générateur pour maintenir le milieu à la température désirée.
Les rapports molaires par rapport au DCNB sont les suivants :
DCNB = 1
Solvant adiponitrile (ADN)=3
CsF =2, 1

| Solvant | Temp. (°C) | Temps (min) | TT (%) | RR CFNB (%) | RR DFNB (%) | □ RT (%) |
|---|---|---|---|---|---|---|
| ADN | 180°C | 10mn | 51 | 43 | 8 | 100 |
| ADN | 200°C | 10mn | 72 | 50 | 22 | 100 |
| ADN | 220°C | 10mn | 96 | 32 | 54 | 90 |

## Revendications

1. Utilisation comme solvant aprotique polaire dans les réactions de substitution nucléophile d'un nitrile dont la masse moléculaire est supérieure à 79, de préférence supérieure à 90 et **caractérisé par le fait que** ledit nitrile comporte au moins une fonction nitrile portée par un carbone aliphatique (c'est-à-dire d'hybridation sp³) avec la condition que lorsque les carbones aliphatiques portent, outre une fonction nitrile, une fonction électroattractrice dont la constante de Hammett σₚ est supérieure ou égale à 0,05, ils ne portent pas d'hydrogène.

2. Utilisation selon la revendication 1, **caractérisée par le fait que** lorsque le nitrile présente 2 fonctions électroattractrices (y compris le nitrile), ces 2 fonctions électroattractrices seront séparées par au moins 2 atomes de carbone, avantageusement 3.

3. Utilisation selon la revendication 2, **caractérisée par le fait que** les deux fonctions électroattractrices sont des nitriles.

4. Utilisation selon les revendications 1 à 3, **caractérisée par le fait que** lorsque le nitrile présente 2 fonctions électroattractrices (y compris le nitrile), ces 2 fonctions électroattractrices seront séparées par 4 atomes de carbone.

5. Utilisation selon les revendications 1 à 4, **caractérisée par le fait que** lorsque les carbones aliphatiques portent, outre une fonction nitrile, une fonction électroattractrice dont la constante de Hammett σₚ est supérieure ou égale à 0,2, ils ne portent pas d'hydrogène.

6. Utilisation selon les revendications 1 à 5, **caractérisée par le fait que** le nombre d'hydrogène en alpha d'un groupe électroattracteur de σₚ supérieur ou égal à 0,4 est tel que le rapport H_{α}/H_{T} est au plus égal à 2/3, avantageusement à 1/2, de préférence à 40% et plus préférentiellement à 30%.

7. Utilisation selon les revendications 1 à 6, **caractérisée par le fait que** la masse moléculaire ramenée au nombre de fonctions nitriles aliphatiques est au moins égale à 40, avantageusement à 47, de préférence à 54.

8. Utilisation selon les revendications 1 à 7, **caractérisée par le fait que** la réaction de substitution nucléophile est menée sous activation par micro-ondes.

9. Utilisation selon les revendications 1 à 7, **caractérisée par le fait que** la réaction de substitution nucléophile est une réaction de substitution aromatique.

10. Utilisation selon les revendications 1 à 8, **caractérisée par le fait que** la réaction de substitution nucléophile est une réaction de substitution aromatique d'échange chlore/fluor.

11. Utilisation selon les revendications 1 à 8, **caractérisée par le fait que** la réaction de substitution nucléophile est une réaction de substitution aromatique d'échange chlore/fluor en présence de fluorure de césium.

12. Composition utile comme réactif d'échange chlore/fluor, **caractérisée par le fait qu'**elle comporte un nitrile tel que défini dans les revendications 1 à 5, un agent source d'ions de fluorure choisi parmi les fluorures alcalins ou alcalino-terreux, et éventuellement un agent de transfert de phase avec la condition que lorsque la concentration en agent de transfert de phase est inférieure à 0,5% en masse du nitrile, ce dernier représente au moins 50%, de préférence les 2/3 en masse de la masse des solvants utilisés.

13. Composition selon la revendication 12, **caractérisée par le fait qu'**elle comporte comme agent de transfert de phase du césium.

14. Composition selon les revendications 12 et 13, **caractérisée par le fait qu'**elle comporte en majorité (≥ 50% en équivalent fluor) comme fluorure alcalin, du fluorure de césium.

15. Composition selon les revendications 12 à 14, **caractérisée par le fait que** ledit nitrile est choisi parmi les dinitriles aliphatique.

16. Composition selon les revendications 12 à 14, **caractérisée par le fait que** les nitriles sont choisis parmi le pentanenitrile, l'hexanenitrile, l'octanenitrile, le glutaronitrile, le méthylglutaronitrile, l'adiponitrile, le pimélonitrile, le subéronitrile.

## Patentansprüche

1. Verwendung als polares aprotisches Lösungsmittel bei nukleophilen Substitutionsreaktionen eines Nitrils, dessen Molekulargewicht höher als 79, vorzugsweise höher als 90, ist und **dadurch gekennzeichnet, dass** besagtes Nitril wenigstens eine von einem aliphatischen Kohlenstoff (d.h. mit sp³-Hybridisierung) getragene Nitrilfunktion aufweist, unter der Maßgabe, dass, wenn die aliphatischen Kohlenstoffe außer einer Nitrilfunktion eine Elektronen anziehende Funktion, deren Hammett-σₚ-Konstante höher oder gleich 0,05 ist, tragen, sie keinen Wasserstoff tragen.

2. Verwendung gemäß Anspruch 1, **dadurch gekennzeichnet, dass**, wenn das Nitril 2 Elektronen anziehende Funktionen (worunter das Nitril umfasst ist) trägt, diese 2 Elektronen anziehenden Funktionen durch zumindest 2 Kohlenstoffatome, vorzugsweise 3, getrennt sind.

3. Verwendung gemäß Anspruch 2, **dadurch gekennzeichnet, dass** die 2 Elektronen anziehenden Funktionen Nitrile sind.

4. Verwendung gemäß den Ansprüchen 1 bis 3, **dadurch gekennzeichnet, dass**, wenn das Nitril 2 Elektronen anziehende Funktionen (wovon das Nitril umfasst ist) aufweist, diese 2 Elektronen anziehenden Funktionen durch 4 Kohlenstoffatome getrennt sind.

5. Verwendung gemäß den Ansprüchen 1 bis 4, **dadurch gekennzeichnet, dass**, wenn die aliphatischen Kohlenstoffe außer einer Nitrilfunktion eine Elektronen anziehende Funktion tragen, deren Hammett-σₚ-Konstante größer oder gleich 0,2 ist, sie keinen Wasserstoff tragen.

6. Verwendung gemäß den Ansprüchen 1 bis 5, **dadurch gekennzeichnet, dass** die Anzahl an Wasserstoff in α-Stellung einer Elektronen anziehenden Gruppe von σₚ größer oder gleich 0,4 derart ist, dass das Verhältnis H_{α}/H_{T} höchstens 2/3, vorteilhaft höchstens 1/2 , und vorzugsweise höchstens 40%, und noch bevorzugter höchstens 30% beträgt.

7. Verwendung gemäß den Ansprüchen 1 bis 6, **dadurch gekennzeichnet, dass** das auf die aliphatischen Nitrilfunktionen gerichtete Molekulargewicht wenigstens 40, vorteilhaft 47, vorzugsweise 54, beträgt.

8. Verwendung gemäß den Ansprüchen 1 bis 7, **dadurch gekennzeichnet, dass** die nukleophile Substitutionsreaktion unter Aktivierung durch Mikrowellen durchgeführt wird.

9. Verwendung gemäß den Ansprüchen 1 bis 7, **dadurch gekennzeichnet, dass** die nukleophile Substitutionsreaktion eine aromatische Substitutionsreaktion ist.

10. Verwendung gemäß den Ansprüchen 1 bis 8, **dadurch gekennzeichnet, dass** die nukleophile Substitutionsreaktion eine aromatische Substitutionsreaktion mit Chlor/Fluor-Austausch ist.

11. Verwendung gemäß den Ansprüchen 1 bis 8, **dadurch gekennzeichnet, dass** die nukleophile Substitutionsreaktion eine aromatische Substitutionsreaktion mit Chlor/Fluor-Austausch in Gegenwart von Cäsiumfluorid ist.

12. Als Reagenz für den Chlor/Fluor-Austausch geeignete Zusammensetzung, dadurch gekennnzeichnet, dass sie ein Nitril, wie in den Ansprüchen 1 bis 5 definiert, eine Quelle für Fluoridionen, ausgewählt unter den Alkali- oder Erdalkalifluoriden, und gegebenenfalls ein Phasentransfermittel enthält, unter der Maßgabe, dass, wenn die Konzentration an Phasentransfermittel geringer als 0,5% in der Masse des Nitrils ist, dieses letztere wenigstens 50%, vorzugsweise 2/3, ausgedrückt in Masse der Masse der verwendeten Lösungmittel, ausmacht.

13. Zusammensetzung gemäß Anspruch 12, **dadurch gekennzeichnet, dass** sie als Phasentransfermittel Cäsium enthält.

14. Zusammensetzung gemäß den Ansprüchen 12 und 13, **dadurch gekennzeichnet, dass** sie überwiegend (≥ 50% an Fluoräquivalent) als Alkalifluorid Cäsiumfluorid enthält.

15. Zusammensetzung gemäß den Ansprüchen 12 bis 14, **dadurch gekennzeichnet, dass** das besagte Nitril unter den aliphatischen Dinitrilen ausgewählt wird.

16. Zusammensetzung gemäß den Ansprüchen 12 bis 14, **dadurch gekennzeichnet, dass** die Nitrile unter Pentannitril, Hexannitril, Octannitril, Glutaronitril, Methylglutaronitril, Adiponitril, Pimelonitril, Suberonitril ausgewählt sind.

## Claims

1. Use as polar aprotic solvent in nucleophilic substitution reactions of a nitrile with a molecular mass of greater than 79, preferably of greater than 90, and **characterized in that** said nitrile comprises at least one nitrile functional group carried by an aliphatic carbon (that is to say, with sp³ hybridization), with the condition that, when the aliphatic carbons carry, in addition to a nitrile functional group, an electron-withdrawing functional group with a Hammett constant σₚ of greater than or equal to 0.05, they do not carry hydrogen.

2. Use according to Claim 1, **characterized in that**, when the nitrile exhibits 2 electron-withdrawing functional groups (including the nitrile), these 2 electron-withdrawing functional groups will be separated by at least 2 carbon atoms, advantageously 3.

3. Use according to Claim 2, **characterized in that** the two electron-withdrawing functional groups are nitriles.

4. Use according to Claims 1 to 3, **characterized in that**, when the nitrile exhibits 2 electron-withdrawing functional groups (including the nitrile), these 2 electron-withdrawing functional groups will be separated by 4 carbon atoms.

5. Use according to Claims 1 to 4, **characterized in that**, when the aliphatic carbons carry, in addition to a nitrile functional group, an electron-withdrawing functional group with a Hammett constant σₚ of greater than or equal to 0.2, they do not carry hydrogen.

6. Use according to Claims 1 to 5, **characterized in that** the number of hydrogens in the alpha position with respect to an electron-withdrawing group with σₚ greater than or equal to 0.4 is such that the H_{α}/H_{T} ratio is at most equal to 2/3, advantageously to 1/2, preferably to 40% and more preferably to 30%.

7. Use according to Claims 1 to 6, **characterized in that** the molecular mass, with respect to the number of aliphatic nitrile functional groups, is at least equal to 40, advantageously to 47, preferably to 54.

8. Use according to Claims 1 to 7, **characterized in that** the nucleophilic substitution reaction is carried out under activation by microwaves.

9. Use according to Claims 1 to 7, **characterized in that** the nucleophilic substitution reaction is an aromatic substitution reaction.

10. Use according to Claims 1 to 8, **characterized in that** the nucleophilic substitution reaction is a chlorine/fluorine exchange aromatic substitution reaction.

11. Use according to Claims 1 to 8, **characterized in that** the nucleophilic substitution reaction is a chlorine/fluorine exchange aromatic substitution reaction in the presence of caesium fluoride.

12. Composition of use as chlorine/fluorine exchange reactant, **characterized in that** it comprises a nitrile as defined in Claims 1 to 5, an agent which is a source of fluoride ions chosen from alkali metal fluorides or alkaline earth metal fluorides, and optionally a phase transfer agent, with the condition that, when the concentration of phase transfer agent is less than 0.5% by mass of the nitrile, the latter represents at least 50%, preferably 2/3, by mass of the mass of the solvents used.

13. Composition according to Claim 12, **characterized in that** it comprises caesium as phase transfer agent.

14. Composition according to Claims 12 and 13, **characterized in that** it predominantly comprises (≥ 50% as fluorine equivalent) caesium fluoride as alkali metal fluoride.

15. Composition according to Claims 12 to 14, **characterized in that** said nitrile is chosen from aliphatic dinitriles.

16. Composition according to Claims 12 to 14, **characterized in that** the nitriles are chosen from pentanenitrile, hexanenitrile, octanenitrile, glutaronitrile, methylglutaronitrile, adiponitrile, pimelonitrile or suberonitrile.
